# EUROPEAN PATENT APPLICATION

(11) **EP 2 642 292 A1**
(43) Date of publication of application: **25.09.2013**
(21) Application number: 11841158.6
(22) Date of filing: 16.11.2011
(51) Int. Cl.: G01N 33/68, G01N 33/15, G01N 33/50, G01N 33/53

(54) **METHOD FOR TESTING FOR CEREBRAL INFARCTION VIA CARTILAGE ACIDIC PROTEIN 1**

(30) Priority: 16.11.2010 JP 2010255932
(71) Applicant: Mitsubishi Chemical Corporation, Chiyoda-ku Tokyo 100-8251 (JP); Kyushu University National University Corporation, Higashi-ku Fukuoka-shi Fukuoka 812-8581 (JP); Hisayama Research Institute For Lifestyle Diseases, Kasuya-gun, Fukuoka 811-2501 (JP)
(72) Inventor: KITAZONO, Takanari, Fukuoka-shi Fukuoka 812-8581 (JP); KAMOUCHI, Masahiro, Fukuoka-shi Fukuoka 812-8581 (JP); AGOU, Tetsurou, Fukuoka-shi Fukuoka 812-8581 (JP); KUWASHIRO, Takahiro, Fukuoka-shi Fukuoka 812-8581 (JP); KIYOHARA, Yutaka, Fukuoka-shi Fukuoka 812-8581 (JP); HATA, Jun, Fukuoka-shi Fukuoka 812-8581 (JP); AWANO, Hideto, Osaka-shi Osaka 541-8505 (JP); KOBAYASHI, Teruaki, Osaka-shi Osaka 532-8505 (JP)
(74) Representative: Vossius & Partner
(86) International application number: PCT/JP2011/076415
(87) International publication number: WO 2012/067151

(57) **Abstract**

A test method for diagnosing cerebral infarction, a test method for diagnosing the disease type of cerebral infarction, and a test method for predicting prognosis of cerebral infarction in a subject animal, which test methods comprise the step of measuring the amount of cartilage acidic protein 1 in a blood sample collected from the animal.

## Description

### TECHNICAL FIELD

The present invention relates to: a method for testing for cerebral infarction; method for evaluating a therapeutic or prophylactic effect on cerebral infarction; method for screening a prophylactic drug or therapeutic drug for cerebral infarction; and a kit for testing for cerebral infarction, for evaluating a therapeutic effect on cerebral infarction, or for screening a candidate compound for a prophylactic/therapeutic drug for cerebral infarction.

### BACKGROUND ART

Stroke is a general term for cerebrovascular diseases such as cerebral infarction, cerebral hemorrhage and subarachnoid hemorrhage. Among these types of stroke, cerebral infarction is recently relatively increasing. Examples of pharmacotherapies for cerebral infarction during the acute phase include thrombolysis, anticoagulant therapy, antiplatelet therapy and administration of a cerebroprotective agent, and surgery is carried out for seriously ill patients and patients with hemorrhagic cerebral infarction. The symptoms of cerebral infarction are most severe during the acute phase, and gradually ameliorated thereafter. This is due to swelling of a necrotic brain tissue which compresses and damages brain tissues surrounding it. As the swelling subsides, functions of the surrounding tissues are recovered, and the symptoms become fixed. However, since free radicals released from the site of brain ischemia have a function to cause necrosis of the surrounding tissues, it is said that continuous treatment is necessary for improvement of the functional prognosis even after the acute phase.

Examples of the clinical type of cerebral infarction include lacunar infarction, which occurs due to occlusion of a small cerebral artery; atherothrombotic cerebral infarction, which occurs due to occlusion of a large cerebral artery caused by an atheroma; and cardiogenic brain embolism, which occurs due to occlusion of a cerebral artery caused by an embolus derived from a thrombus in the heart. Since appropriate therapeutic methods during the acute phase and the chronic phase are different among the clinical types, a method that enables rapid diagnosis of the clinical type of cerebral infarction in a patient has been demanded.
Examples of known methods for classifying the clinical type of cerebral infarction include a method wherein observation of clinical symptoms, echocardiography, MRI, MRA, cervical vascular ultrasonography and/or the like is/are carried out and the type is classified based on a flow chart for clinical diagnosis of cerebral infarction according to the TOAST classification (Non-patent Document 1) or the like, and a method using molecular markers wherein, more specifically, molecular markers such as CRP, D-Dimer, RAGE, MMP-9, S100B, BNP and/or the like are simultaneously measured within 24 hours after onset of cerebral infarction and specific cut-off values are set for BNP and D-Dimer to enable differential diagnosis of cardiogenic brain embolism from other disease types (Non-patent Document 2). However, molecular markers that enable more rapid and more accurate diagnosis of the disease type have been demanded.
The prognosis of cerebral infarction is judged by evaluating the degree of disability in daily performance based on the modified Rankin Scale (Non-patent Document 3) or Barthel Index (Non-patent Document 4) between 3 months after the onset and 1 year after the onset. Prediction of the prognosis in advance is important for early determination of the therapeutic method for cerebral infarction. It has been disclosed that death can be predicted by determining a specific cut-off value for BNP in the blood plasma measured on admission (Non-patent Document 5), but molecular markers that predict the degree of disability in patients who survived have been demanded.
The extent of symptoms (severity) of cerebral infarction is judged by evaluating the consciousness disorder, movement disorder, higher function disorder and the like based on the National Institute of Health Stroke Scale (NIHSS) between immediately after the onset and 3 months after the onset. Considerable exacerbation occurs in symptoms of cerebral infarction during hospitalization, and whether or not the cerebral infarction is progressive can be confirmed by observation of changes in NIHSS. Since progressive cerebral infarction is likely to be associated with a poor prognosis, its early prediction is important for satisfactory treatment. However, the method for predicting progressive cerebral infarction has not been established, and molecular markers for such prediction have been demanded.
Patent Document 1 describes use of cartilage acidic protein 1 as a marker for cardiovascular diseases, blood diseases, nervous diseases, respiratory diseases, digestive diseases and urologic diseases. However, the document only shows that the expression level of cartilage acidic protein 1 is higher in model animals for ischemia/myocardial infarction, and does not show any particular data indicating that cartilage acidic protein 1 can be used for diagnosis of these diseases.

### PRIOR ART DOCUMENTS

### [Patent Document]

Patent Document 1: US 2010/0081136 A

### [Non-patent Documents]

Non-patent Document 1: Lee, L. J. et al., Stroke, 1081-1089 (2000)
Non-patent Document 2: Montaner et al., Stroke, 2280-2287(2008)
Non-patent Document 3: van Swieten, J. C et al., Stroke, 604-607 (1988)
Non-patent Document 4: Mahoney, F. L et al., Maryland State Med J, 61-65 (1965)
Non-patent Document 5: Shibazaki, K et al., Inter Med, 1601-1604 (2009)
Non-patent Document 6: Brott T. et al., Stroke, 20, 864 (1989)

### SUMMARY OF THE INVENTION

In view of the above problems, the present invention aims to provide a rapid and accurate method for testing for cerebral infarction wherein serum, blood plasma or the like from a patient with cerebral infarction or the like is used, and a method for evaluating a therapeutic effect on cerebral infarction.

In order to solve the problems described above, the present inventors intensively studied to discover that the plasma concentration of cartilage acidic protein 1 is different among healthy individuals and patients with various disease types and prognoses, and that the amount of cartilage acidic protein 1 can be a useful index for testing for cerebral infarction, thereby completed the present invention.

The gist of the present invention is as follows.
[1] A test method for diagnosing cerebral infarction in a subject animal, comprising the step of measuring the amount of cartilage acidic protein 1 in a blood sample collected from said animal, wherein, in cases where the amount of said protein is lower than the amount in a healthy individual, said animal is judged to be cerebral infarction.
[2] The test method for diagnosing cerebral infarction according to [1], wherein said blood sample collected from said subject animal is a blood sample collected from said subject animal at any period between immediately after onset and 20 days after onset of cerebral infarction-like symptoms.
[3] A test method for diagnosing the disease type of cerebral infarction in a subject animal, said method comprising the step of measuring the amount of cartilage acidic protein 1 in a blood sample collected from said animal.
[4] The test method according to [3], wherein said diagnosis of the disease type is for diagnosing any of lacunar infarction, atherothrombotic, cardiogenic brain embolism, aortogenic brain embolism, branch atheromatous disease, arterial dissection and unclassified type.
[5] A test method for predicting prognosis of cerebral infarction in a subject animal, said method comprising the step of measuring the amount of cartilage acidic protein 1 in a blood sample collected from said animal.
[6] The test method for predicting prognosis of cerebral infarction according to [5], wherein said blood sample collected from said subject animal is a blood sample collected from said subject animal 7 days after onset of cerebral infarction.
[7] A test method for predicting progressiveness of cerebral infarction in a subject animal, said method comprising the step of measuring the amount of cartilage acidic protein 1 in a blood sample collected from said animal.
[8] The test method for predicting progressiveness of cerebral infarction according to [7], wherein said blood sample collected from said subject animal is a blood sample collected from said subject animal 7 days after onset of cerebral infarction.
[9] A method for evaluating a prophylactic or therapeutic effect on cerebral infarction in an animal in need of prophylaxis or therapy of cerebral infarction, said method comprising the step of measuring the amount of cartilage acidic protein 1 in a blood sample collected from said animal to which a prophylactic drug or therapeutic drug for cerebral infarction was administered.
[10] A method for screening a prophylactic drug or therapeutic drug for cerebral infarction, said method comprising the step of contacting a subject with a candidate compound for prophylactic drug or therapeutic drug for cerebral infarction and then measuring the amount of cartilage acidic protein 1 in said subject.
[11] A kit for testing for cerebral infarction, for evaluating a therapeutic effect on cerebral infarction, or for screening a candidate compound for a prophylactic/therapeutic drug for cerebral infarction, said kit comprising a reagent which enables measurement of the amount of cartilage acidic protein 1.

By the present invention, cartilage acidic protein 1, whose plasma concentration is different among healthy individuals and cerebral infarction patients with various disease types, prognoses and progressiveness, is provided as a molecular marker for testing for cerebral infarction (which may be referred to as "CRTAC1 protein marker" in the present description). By using the CRTAC1 protein marker alone or in combination with another/other molecular marker(s) for cerebral infarction, cerebral infarction can be rapidly and accurately diagnosed, or classification of the disease type and prediction of the prognosis of cerebral infarction can be carried out.

### EMBODIMENTS FOR CARRYING OUT THE INVENTION

Embodiments of the present invention are described below in detail.

### (1) Testing Method for Cerebral Infarction

The first embodiment of the present invention is a method for testing a subject animal for cerebral infarction, which method comprises the step of measuring the amount of cartilage acidic protein 1 in a blood sample collected from the animal.

In the present description, "cerebral infarction" is a disease wherein a cerebral artery is occluded by a thrombus or embolus to cause ischemia, leading to necrosis and disorder of brain tissues. The cerebral infarction includes any of: lacunar infarction, which occurs due to occlusion of a small cerebral artery; atherothrombotic cerebral infarction, which occurs due to occlusion of a large cerebral artery caused by an atheroma; cardiogenic brain embolism, which occurs due to occlusion of a cerebral artery caused by an embolus derived from a thrombus in the heart; aortogenic brain embolism, which is an embolism caused by occlusion of a cerebral artery by an embolus dissociated from an atherosclerotic lesion formed at a site between the ascending aorta and aortic arch; "branch atheromatous disease" (which may be hereinafter referred to as "BAD"), which is perforating branch infarction caused by occlusion of the inlet area of a perforating branch by a plaque formed in a major artery; and arterial dissection, which occurs due to occlusion of a cerebral blood vessel by invasion of blood into the arterial wall due to damaging of the intima of the cerebral blood vessel. The cerebral infarction includes any of the acute phase, subacute phase, convalescent phase (chronic phase) and the like.

In the present description, cartilage acidic protein 1 (which may be hereinafter referred to as "CRTAC1 protein") means cartilage acidic protein 1 as the object whose content in a blood sample is measured in the test method of the present invention. The cartilage acidic protein is not limited as long as it is derived from a subject animal, and specific examples of the protein derived from human include the protein indicated by a specific amino acid sequence described below. The protein also includes fragments, derivatives and mutants of such proteins having similar functions.

In the test method described above, the "subject animal" is not limited as long as there is a possibility of onset of cerebral infarction therein, and specific examples of the subject animal include human, monkey, and rodents such as rat. The test method of the present invention for cerebral infarction is especially preferably applied to human patients with suspected cerebral infarction, human patients who developed cerebral infarction, and the like.

The "blood sample" collected from a subject animal is not limited as long as it contains the CRTAC1 protein and the concentration of the protein can be measured, and specific examples of the blood sample include blood plasma such as EDTA plasma and citrate plasma; serum; and whole blood. Among these, EDTA plasma is preferably used since it can be easily collected and preserved, and can be collected in a large amount. The timing of collection of a blood sample from the subject animal may be any timing as long as it is timing when diagnosis of cerebral infarction is to be carried out. Since the symptoms and pathological conditions of cerebral infarction change from moment to moment, a detailed description will be given below.

In the test method of the present invention, the cartilage acidic protein 1 as the object whose content in a blood sample collected from a subject animal is measured is, in cases of human, a protein having the amino acid sequence shown in UniProtKB with the entry name CRAC1_HUMAN. The sequence information can be obtained by, for example, entering the above entry name in such a database as National Center for Biotechnology Information (NCBI). The above amino acid sequence is for human, and, in cases where the subject animal is not human, a homologue protein derived from the animal is the protein to be subjected to the measurement.

The method for measuring the content of the above protein in a blood sample is not limited as long as the content of the protein can be measured thereby, and examples of the method include known immunoassays using an antibody against the protein; method by combination of a chromatography technique with time-of-flight mass spectrometry (TOF-MS) wherein the mass of all the components captured by a chromatography carrier (e.g., cation exchanger, anion exchanger, hydrophobic chromatography carrier or metal ion) under predetermined conditions is measured as a whole; and two-dimensional gel electrophoresis. Preferred examples of the immunoassays include a method wherein quantitative detection is carried out by enzyme immunoassay, and a method wherein measurement is carried out by fluorescence immunoassay or chemiluminescent immunoassay. The enzyme immunoassay, among the labeled immunoassays, is a detection method using an enzyme as a labeling substance. Enzyme-linked immunosorbent assay (ELISA), which uses an immunosorbent, is especially preferably selected. Further, among ELISA methods, the sandwich method can be one of especially preferred measurement methods in view of simplicity of operation, economical convenience and, especially, versatility in clinical tests. These measurement methods may be carried out according to methods described in general laboratory manuals such as New Biochemistry Experiments Lecture (Japanese Biochemical Society (ed.), Tokyo Kagaku-dojin); Molecular Cloning, A Laboratory Manual (T. Maniatis et al., Cold Spring Harbor Laboratory (2001)); and Antibodies-A Laboratory Manual (E. Harlow et al., Cold Spring Harbor Laboratory (1988)); and methods similar to these.

The antibody or the like (including an antibody fragment) to be used for carrying out the measurement can be obtained by a known method using the CRTAC1 protein as an antigen. However, the antibody or the like does not need to be one produced using the CRTAC1 protein as an antigen, and is not limited as long as it at least shows cross reactivity with the protein and its content can be measured. Preferred examples of such immunoassay and an antibody used therefor include anti-human CRTAC1 antibody manufactured by R&D (catalogue No. AF5234).

The blood sample in the method of the present invention is preferably used for the above measurement immediately after collection from the subject animal, but a preserved blood sample may also be used. The method for preserving the blood sample is not limited as long as it is carried out under conditions where the amount of the molecular marker for cerebral infarction does not change, and the method is preferably carried out under a low-temperature condition at 0 to 10°C which does not cause freezing, under dark and non-vibrating conditions. In cases where freezing is necessarily carried out, the method is preferably one wherein degradation, oxidation reaction and the like of the marker molecule can be avoided.

In the test method of the present invention, the content of the CRTAC1 protein marker in a blood sample collected from the subject animal (this may be referred to as "sample" in the present description) may be measured, and this may be used as an index for testing for cerebral infarction. Further, more accurate testing is possible by combining the method of the present invention with measurement of the contents of known cerebral infarction markers such as CRP and D-Dimer in the sample, or with use of a composite index wherein observation of clinical symptoms is combined with results of echocardiography, MRI, MRA, cervical vascular ultrasonography and/or the like.

In cases where the cerebral infarction test is carried out using as an index the content of the molecular marker for a cerebral infarction, the absolute value of the content of the molecular marker for cerebral infarction in the sample may be compared with that in a healthy individual (healthy animal), or the test may be carried out by a method wherein an appropriate cut-off value is defined. The content of the marker for cerebral infarction in a blood sample from a healthy individual can be obtained by preliminarily collecting blood from a healthy individual who was clinically confirmed to be free of cerebral infarction and quantifying the content by carrying out the same treatment and measurement as in the case of the blood collected from the subject animal. The method for clinically confirming various types of cerebral infarction is not limited, and examples of the method include diagnosis methods using an apparatus such as cranial X-ray CT, cranial MRI, MRA, cerebral angiography or cervical vascular ultrasonography.

The cut-off value means a value defined for distinguishing between a group with a disease of interest and a nondiseased group (group which is free of the disease of interest), focusing on a specific substance. When the diseased group and the nondiseased group are to be distinguished from each other for the disease of interest, each individual can be judged to be negative for the disease in cases where the measured value is not higher than a cut-off value and to be positive for the disease in cases where the measured value is not lower than the cut-off value, or can be judged to be positive for the disease in cases where the measured value is not higher than a cut-off value and to be negative for the disease in cases where the measured value is not lower than the cut-off value (Masamitsu Kanai (ed.), Clinical Test Handbook, Kanehara & Co., Ltd.).
Examples of the index to be used for the purpose of evaluating clinical usefulness of the cut-off value include the sensitivity and the specificity. When a population is assessed using a cut-off value, the sensitivity (true-positive rate) can be represented by the value of a/(a+b) and the specificity (true-negative rate) can be represented by the value of d/(c+d), wherein in these formulae, a represents the number of individuals who are patients with the disease and were judged to be positive (true positive); b represents the number of individuals who are patients with the disease but were judged to be negative (false negative); c represents the number of individuals who are not patients with the disease but were judged to be positive (false positive); and d represents the number of individuals who are not patients with the disease and judged to be negative (true negative).

Between the diseased group and the nondiseased group of the disease of interest, the distributions of measured values usually partially overlap with each other. Therefore, by raising or lowering the cut-off value, the sensitivity and the specificity can be changed. When the cut-off value is lowered, the sensitivity increases but the specificity decreases; and when the cut-off value is raised, the sensitivity decreases but the specificity increases. In a judging method, it is preferable that both the sensitivity and the specificity are higher. A judging method wherein the values of the sensitivity and the specificity do not exceed 0.5 cannot be said to be useful.

Examples of the method for setting a cut-off value include a method wherein a value between the center and either end is set as the cut-off value such that 95% of the distribution of the nondiseased group is contained, and a method wherein, in cases where the distribution of the nondiseased group shows a normal distribution, the mean + 2 standard deviations (SDs) or the mean - 2SDs is set as the cut-off value.

Further, in general, since the sensitivity and the specificity fluctuate depending on the cut-off value set as described above, the judgment of whether a diagnosis method is useful is preferably carried out based on evaluation with an index that keeps the sensitivity and the specificity high when the cut-off value is raised or lowered, such as the AUC value of the ROC curve, rather than simply carrying out evaluation with the sensitivity and the specificity based on a specific cut-off value. The AUC value of the ROC curve is 1 when there is a cut-off value that makes both sensitivity and specificity 100%, while the AUC value is close to 0.5 when the diagnostic performance is not good. Therefore, when the performance of a diagnostic method is judged based on the AUC value of the ROC curve, the method can be evaluated as being appropriate as a diagnostic method if the value is not less than 0.7. Since the CRTAC1 protein marker described in the present invention also had an AUC value of the ROC curve of not less than 0.7, the marker can be used for the later-mentioned test methods for cerebral infarction.

### (1-1) Test Method for Cerebral Infarction Using Cartilage Acidic Protein 1 (CRTAC1) Marker

Examples of specific methods for carrying out the test method for cerebral infarction using the CRTAC1 protein marker of the present invention include a test method for distinguishing between a healthy individual and a cerebral infarction patient, that is, a test method wherein the amount of the CRTAC1 protein in a blood sample collected from a subject animal is measured and, in cases where the amount of the CRTAC1 protein is lower than that in a healthy individual, the subject animal is judged to be suffering from cerebral infarction. The timing of collection of the sample in this case is not limited as long as it is immediately after the onset of cerebral infarction-like symptoms or later, and is preferably between immediately after the onset and 20 days after onset, in view of carrying out diagnosis. The especially preferred timing is between immediately after the onset and 9 days after onset. The content of the CRTAC1 protein marker in the collected sample may be measured and its absolute value may be compared with that in a healthy individual (healthy animal), or an appropriate cut-off value may be set by the above-described method and a test may be carried out using it. In cases where the absolute value of the content of the CRTAC1 protein marker in a blood sample collected from a subject with suspected cerebral infarction is higher than that of a healthy individual (healthy animal), or higher than a cut-off value, the subject can be judged to be a cerebral infarction patient. The content of the marker for cerebral infarction in the blood sample from a healthy individual can be obtained by preliminarily collecting blood from a healthy individual who was clinically confirmed to be free of cerebral infarction and quantifying the content by carrying out the same treatment and measurement as in the case of the blood collected from the subject animal. The method for clinically confirming various types of cerebral infarction is not limited, and examples of the method include diagnosis methods using an apparatus such as cranial X-ray CT, cranial MRI, MRA, cerebral angiography or cervical vascular ultrasonography. Further, more accurate testing is possible by combining the method of the present invention with measurement of the contents of known cerebral infarction markers such as CRP and D-Dimer in the sample, or with use of a composite index wherein observation of clinical symptoms is combined with results of echocardiography, MRI, MRA, cervical vascular ultrasonography and/or the like.

### (1-2) Method for Testing Disease Type of Cerebral Infarction Using CRTAC1 Protein Marker

Examples of specific methods for carrying out the test method for cerebral infarction using the CRTAC1 protein marker of the present invention include a test method for distinguishing among disease types of cerebral infarction. The disease types mean per se known disease types which are clinically distinguished, and specific examples of the disease types include "lacunar infarction" (which may be referred to as "lacunar" in the present description); "atherothrombotic cerebral infarction" (which may be referred to as "atherothrombotic " in the present description); "cardiogenic brain embolism" (which may be referred to as "cardiogenic brain embolus" in the present description); "aortogenic brain embolism", which is an embolism caused by occlusion of a cerebral artery by an embolus dissociated from an atherosclerotic lesion formed at a site between the ascending aorta and aortic arch; "branch atheromatous disease" (which may be hereinafter referred to as "BAD"), which is perforating branch infarction caused by occlusion of the inlet area of a perforating branch by a plaque formed in a major artery; arterial dissection, which occurs due to occlusion of a cerebral blood vessel by invasion of blood into the arterial wall due to damaging of the intima of the cerebral blood vessel; and disease types that cannot be categorized into any of these (which may be hereinafter referred to as "unclassified type").

In order to distinguish among disease types of cerebral infarction by measuring the amount of the CRTAC1 protein contained in a sample, cut-off values of the amount of the CRTAC1 protein in the blood sample, with which the disease types can be distinguished, are determined by the above-described method, and the amount of the CRTAC1 protein in the sample is compared with the cut-off values for judging the disease type of the cerebral infarction.

The disease types which can be distinguished based on the amount of the CRTAC1 protein contained in the blood sample of the present invention are not limited as long as a cut-off value can be set such that those disease type groups can be distinguished from each other based on the content of the CRTAC1 protein. Specific examples of such disease types include, as shown in Table 1, "atherothrombotic cerebral infarction" and "arterial dissection"; "cardiogenic brain embolism" and "arterial dissection"; "lacunar infarction" and "BAD"; "lacunar infarction" and "arterial dissection"; "aortogenic brain embolism" and "BAD"; "aortogenic brain embolism" and "arterial dissection"; "BAD" and "arterial dissection"; "BAD" and "unclassified type"; "atherothrombotic cerebral infarction and "lacunar infarction"; "cardiogenic brain embolism" and "lacunar infarction"; "cardiogenic brain embolism" and "BAD"; "atherothrombotic cerebral infarction" and "BAD"; "arterial dissection" and other disease types; and "BAD" and other disease types.

The timing for obtaining the sample for carrying out the measurement is not limited as long as it is immediately after the onset of cerebral infarction-like symptoms or later. Table 1 shows preferred timings.

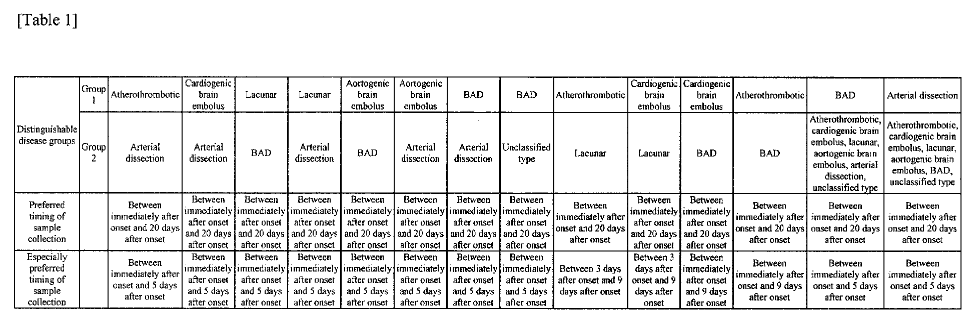

In the measurement method of the present invention, the amount of the CRTAC1 protein in the blood sample is preliminarily measured in patients who were clinically judged as suffering from the above distinguishable disease types, and cut-off values for distinguishing those disease type groups are set by the above method. By measuring the content of the CRTAC 1 protein marker in the collected sample and comparing its absolute value with the cut-off values set by the above method, the disease type of the subject (animal) from which the sample was collected can be judged.

Further, when a test for the above disease types is carried out, more accurate testing is possible by combining the method of the present invention with measurement of the contents of known cerebral infarction markers such as CRP and D-Dimer in the sample, or with use of a composite index wherein observation of clinical symptoms is combined with results of echocardiography, MRI, MRA, cervical vascular ultrasonography and/or the like.

### (1-3) Test Method for Prediction of Prognosis of Cerebral Infarction Using CRTAC1 Protein Marker

Examples of specific methods for carrying out the test method for cerebral infarction using the CRTAC1 protein marker of the present invention include a test method for prediction of the prognosis of cerebral infarction in a subject (animal). The prognosis of cerebral infarction is indicated after a certain period of time following the onset of cerebral infarction in terms of the degree of disability due to cerebral infarction in the patient. The degree of disability can be evaluated using a per se known index which is already established. Example of the index to be used include a Japanese version of the modified Rankin Scale (mRS) criterion (Yukito Shinohara et al., mRS Reliability Study Group, Reliability of modified Rankin Scale-Introduction of a guidance scheme and a questionnaire written in Japanese-, Jpn J Stroke 2007, 29:6-13).

The timing meant by the term "after a certain period of time" is not limited as long as it is after the onset, and the evaluation is generally carried out between, for example, 3 months after the onset and one year after the onset, in view of evaluation of rehabilitation of the subject (animal). In consideration of usefulness of the prediction method, the prediction of prognosis is preferably carried out by, for example, a method wherein the amount of the CRTAC1 protein in a blood sample obtained from the subject between immediately after the onset and about 2 weeks after the onset is used for prediction of the condition of the subject 3 months after the onset.

In the measurement method of the present invention, patients are clinically judged for the above-described mRS at the timing of prediction of interest, and the amount of the CRTAC1 protein in a blood sample of each patient collected at the timing of measurement of interest is measured in advance. Thereafter, between mRS values that should be distinguished at the timing of prediction of interest, a cut-off value for distinguishing the mRS groups is set by the above-described method. More specifically, for example, in cases where a patient with mRS of 0 to 1 and a patient with mRS of 2 to 5 in terms of mRS determined 3 months after the onset are to be distinguished, the amount of the CRTAC1 protein in a blood sample collected 7 days after the onset (the timing of measurement of interest) is measured in patients with mRS of 0 to 5 determined 3 months after the onset (the timing of prediction of interest), and a cut-off value is set between the measured values for patients with mRS of 0 to 1 and the measured values for patients with mRS of 2 to 5 determined 3 months after the onset. Thereafter, a sample is collected at the timing of measurement of interest, and the content of the CRTAC1 protein marker in the sample is measured. By comparing its absolute value with the cut-off value set by the above method, the condition of the subject (animal) at the timing of prediction of interest can be predicted.

Especially preferred examples of the measurement method of the present invention include a method wherein the amount of the CRTAC1 protein in a sample collected from the subject between immediately after the onset and about 2 weeks after the onset, more preferably between immediately after the onset and 9 days after the onset, is measured to predict whether mRS of the subject about 3 months after the onset will be 2 to 5 or not more than 1.

Other preferred examples of the prediction method of the present invention include a method wherein the amount of the CRTAC1 protein in a sample collected from the subject between immediately after the onset and about 2 weeks after the onset, more preferably between immediately after the onset and 9 days after the onset, is measured to predict whether mRS of the subject about 3 months after the onset will be 3 to 5 or not more than 2, and a method wherein the amount of the CRTAC1 protein in a sample collected from the subject between immediately after the onset and about 2 weeks after the onset, more preferably between immediately after the onset and 9 days after the onset, is measured to predict whether mRS of the subject about 3 months after the onset will be 4 to 5 or not more than 3.

By this, the subject (animal) diagnosed with cerebral infarction by the method of the present invention can receive an appropriate therapy for the respective diseases, and hence the prognosis and the therapeutic effect may be very good.

### (1-4) Test Method for Prediction of Progressiveness of Cerebral Infarction Using CRTAC1 Protein Marker

Examples of specific methods for carrying out the test method for cerebral infarction using the CRTAC1 protein marker of the present invention include a test method for prediction of progressiveness of cerebral infarction in a subject (animal). The progressiveness of cerebral infarction is indicated within a certain period of time following the onset of cerebral infarction in terms of the change in the severity of cerebral infarction in the patient. The severity can be evaluated using a per se known index which is already established. Example of the index used include National Institute of Health Stroke Scale (NIHSS) (Brott T. et al., Measurement of acute cerebral infarction: A clinical examination scale. Stroke, 20, 864 (1989)).

The timing meant by the term "within a certain period of time" is not limited as long as it is after the onset, and the evaluation is generally carried out between, for example, immediately after the onset and 2 weeks after the onset, in view of evaluation of the condition of the subject (animal) during hospitalization. In consideration of usefulness of the prediction method, the prediction of progressiveness is preferably carried out by, for example, a method wherein the amount of the CRTAC1 protein in a blood sample collected from the subject between immediately after the onset and about 1 week after the onset is used for prediction of whether or not the disease in the subject is progressive.

In the measurement method of the present invention, patients are clinically judged for the above-described NIHSS at the timing of prediction of interest, and the amount of the CRTAC1 protein in a blood sample of each patient collected at the timing of measurement of interest is measured in advance. Thereafter, for changes in NIHSS that should be distinguished at the timing of prediction of interest, a cut-off value for distinguishing between progressive and nonprogressive cases is set by the above-described method. More specifically, for example, in cases where NIHSS determined within 2 weeks after the onset of cerebral infarction (the timing of prediction of interest) is higher than NIHSS determined immediately after the onset by 1 or more, the disease is defined as being progressive, while in cases where NIHSS did not change, or decreased by 1 or more, the disease is defined as being nonprogressive. The amount of the CRTAC1 protein in each blood sample collected between immediately after the onset and 7 days after the onset (the timing of measurement of interest) is measured to set a cut-off value between the measured values for patients with progressive cerebral infarction and the measured values for patients with nonprogressive cerebral infarction according to NIHSS determined within 2 weeks. Thereafter, a sample is collected at the timing of measurement of interest, and the content of the CRTAC1 protein marker in the sample is measured. By comparing its absolute value with the cut-off value set by the above method, the condition of the subject (animal) at the timing of prediction of interest can be predicted.

Especially preferred examples of the prediction method of the present invention include a method wherein the amount of the CRTAC1 protein in a sample collected from the subject between immediately after the onset and about 2 weeks after the onset, more preferably between immediately after the onset and 9 days after the onset, is measured to predict whether NIHSS of the subject thereafter will increase by 1 or more, or will be the same or decrease by 1 or more, relative to NIHSS determined immediately after the onset of cerebral infarction.

### (2) Kit for Measuring Cerebral Infarction

The present invention also includes a kit to be used for the above cerebral infarction test, and a kit described below for evaluating a prophylactic or therapeutic effect on cerebral infarction or for carrying out a method for screening a prophylactic or therapeutic drug for cerebral infarction. The content of the kit is constituted by a combination of an instrument(s) and/or a reagent(s). A kit is included in the kit of the present invention as long as it contains substances which are essentially the same as the constituents described below or contains substances which are essentially the same as a part thereof, even in cases where the constitution or form is different. As a reagent, an anti-CRTAC1 protein marker antibody is contained in cases where the CRTAC1 protein marker is measured by immunoassay. Further, as required, a diluent for a biological sample, antibody-immobilized solid phase, reaction buffer, washing liquid, labeled secondary antibody or an antibody fragment thereof, reagent for detection of a labeled substance, standard substance and/or the like is/are also contained. Examples of the diluent for a biological sample include an aqueous solution containing a surfactant, buffer, and/or a protein(s) such as BSA and/or casein.

The antibody-immobilized solid phase is a macromolecular material having a shape suitable for the purpose, on which an anti-molecular marker antibody or an antibody fragment thereof is immobilized. Examples of the shape include tubes, beads, plates, particulates such as latex, and sticks; and examples of the material include macromolecular materials such as polystyrene, polycarbonate, polyvinyl toluene, polypropylene, polyethylene, polyvinyl chloride, nylon, polymethacrylate, gelatin, agarose, cellulose, polyethylene terephthalate; glasses; ceramics; and metals. Examples of the method for immobilization of the antibody include known methods such as physical methods, chemical methods, and methods by combination of these. Examples of the antibody-immobilized solid phase include one prepared by immobilizing an antibody, antibody fragment or the like on a 96-well polystyrene microtiter plate for immunoassay by hydrophobic immobilization.

The reaction buffer is not limited as long as it provides a solvent environment that allows a binding reaction between the antibody in the antibody-immobilized solid phase and an antigen in the biological sample. Examples of the reaction buffer include those containing a surfactant, buffer, protein(s) such as BSA and/or casein, antiseptic, stabilizer, reaction accelerator and/or the like.

Examples of the labeled secondary antibody or an antibody fragment thereof include the antibody or antibody fragment to be used in the present invention labeled with a labeling enzyme such as horseradish peroxidase (HRP), bovine intestinal alkaline phosphatase or β-galactosidase, which may be mixed with a buffer, protein(s) such as BSA and/or casein, antiseptic and/or the like.

The reagent for detection of a labeled substance is selected depending on the labeling enzyme. Examples of the reagent for horseradish peroxidase include substrates for absorbance measurement, such as tetramethylbenzidine and o-phenylenediamine; fluorescent substrates such as hydroxyphenyl propionate and hydroxyphenyl acetate; and luminescent substrates such as luminol. Examples of the reagent for alkaline phosphatase include substrates for absorbance measurement, such as 4-nitrophenyl phosphate; and fluorescent substrates such as 4-methylumbelliferyl phosphate.

### (3) Method for Evaluating Prophylactic or Therapeutic Effect on Cerebral Infarction

The second mode of the present invention is a method for evaluating a prophylactic or therapeutic effect on cerebral infarction in an animal to which a prophylactic drug or therapeutic drug for cerebral infarction was administered and which requires prophylaxis or therapy of cerebral infarction, the method comprising the step of measuring the amount of the CRTAC1 marker in a blood sample collected from the animal.

Specific examples of the animal requiring prophylaxis or therapy of cerebral infarction include human and the animals described in (1) showing symptoms of cerebral infarction (these may be simply referred to as "subjects"), and patients diagnosed with cerebral infarction by the method of (1).

The prophylactic or therapeutic drug for cerebral infarction is not limited as long as it is one used as a prophylactic or therapeutic drug for cerebral infarction, and examples of the drug include thrombolytic agents such as urokinase and tissue plasminogen activators; anticoagulants such as heparin; anti-platelet agents such as cyclooxygenase inhibitors, phosphodiesterase inhibitors and thromboxane A2 (TXA2) synthesis inhibitors; and cerebroprotective agents such as free radical scavengers.
The prophylactic or therapeutic effect can be judged to have been exerted in cases where the amount of the CRTAC1 protein marker in a blood sample collected from an animal to which a prophylactic drug or therapeutic drug for cerebral infarction was administered and which requires prophylaxis or therapy of cerebral infarction increased or decreased relative to the amount measured before the administration, or became close to the amount in a control animal which is free of cerebral infarction.
Since the prophylactic or therapeutic drug for treatment of cerebral infarction varies depending on the disease type, an appropriate therapy for a patient can be carried out by performing disease-type diagnosis using the CRTAC1 protein marker. For example, in cases where a patient was diagnosed with lacunar infarction, treatment is performed with a thromboxane A2 (TXA2) synthesis inhibitor, and recurrence of cerebral infarction can be prevented with a cyclooxygenase inhibitor. Further, in cases where the possibility of poor prognosis could be predicted with the marker, a therapy for improving the prognosis can be carried out by combined use of several therapeutic drugs such as a free radical scavenger from an early stage of therapy, or by enhancing rehabilitation. Further, in cases where the possibility of progressive cerebral infarction could be predicted, the progressiveness can be prevented or the degree of progression can be suppressed by enhancing the pharmacotherapy by, for example, combined use of a free radical scavenger with an anticoagulant such as argatroban, anti-platelet agent such as phosphodiesterase inhibitor, and/or the like from an early stage of therapy, in order to prevent the symptoms from becoming severe.

### (4) Method for Screening Prophylactic or Therapeutic Drug for Cerebral Infarction

The third embodiment of the present invention is a method for screening a prophylactic drug or therapeutic drug for cerebral infarction, the method comprising the step of contacting a subject with a candidate compound for the prophylactic drug or therapeutic drug for cerebral infarction and then measuring the amount of the CRTAC1 protein marker in a blood sample from the subject.

The subject is a non-human animal individual, tissue, cell or the like wherein the content of the CRTAC1 protein marker is abnormal as in cases of cerebral infarction conditions. Examples of the animal showing symptoms of cerebral infarction include a cerebral infarction model non-human animal wherein cerebral infarction conditions were formed by a surgical method or the like (Yuji Kuge, Kazuo Minematsu et al. Nylon Monofilament for Intraluminal Middle Cerebral Artery Occlusionin Rats. Stroke, 26, 1655 (1995)). In addition to the subject, an individual, tissue, cell or the like of the same species, wherein the content of the CRTAC1 protein marker is the same as that in the healthy condition (normal), is also preferably used.
If a diseased state similar to that of a specific disease type in human can be constructed in a non-human animal individual with cerebral infarction conditions based on the CRTAC1 protein marker, an experiment system for developing a therapeutic drug for cerebral infarction patients with the specific disease type can be established. Further, by monitoring the prognosis of the non-human animal individual using the marker, it is possible to develop a therapeutic drug effective for the prognosis of human cerebral infarction.

Examples of the candidate compound for the prophylactic or therapeutic drug for cerebral infarction which is to be contacted with the subject (which may be hereinafter referred to as "candidate compound") include peptides, proteins, non-peptide compounds and low-molecular-weight compounds, and each of these compounds may be either a novel compound or known compound. Further, each of these compounds may be in the form of a fermentation product, cell extract, plant extract, animal tissue extract or the like containing the compound.
The dose, administration method, treatment time and the like may be appropriately selected depending on the subject used.

In terms of the method for measuring the content of the CRTAC1 protein marker in the subject after the contact with the candidate compound, a method suitable for the subject may be employed. For example, in cases where the subject is a cell, the subject may be prepared into a cell extract according to a known method, and the prepared cell extract may be used as a sample. Alternatively, the cell may be immobilized on a culture plate or slide glass to provide a sample. For example, in cases where a cell extract is prepared to provide a sample, detection may be carried out by ELISA or the like. In preparation of these samples, a constant number of cells are used in advance for extraction, or a constant amount of purified RNA or extracted protein is used, for accurate comparison and analysis of the value obtained as a result of the detection.
In cases where the amount of the CRTAC1 protein marker in the subject after the contact with the candidate compound increased or decreased relative to the amount before the contact, or became close to the amount in a control subject, the candidate compound can be judged to have a prophylactic or therapeutic effect on cerebral infarction.

### EXAMPLES

The present invention will now be described concretely by way of Examples. However, the present invention is not limited to these Examples.

### Example 1

### Evaluation of Test Method for Distinguishing between Healthy Individual and Cerebral Infarction Patient Using Cartilage Acidic Protein 1 Marker

Blood was collected from cerebral infarction patients and healthy individuals (see Table 2 for the number of patients/individuals) immediately after the onset of cerebral infarction (indicated as "DAY 0" in the table) and 7 days after the onset (indicated as "DAY 7" in the table), and EDTA plasma was obtained therefrom. By immunoassay using anti-human CRTAC1 antibody (catalogue No. AF5234) manufactured by R&D, biotinylated anti-human CRTAC1 antibody (catalogue No., BAF5234) manufactured by R&D and Streptavidin-HRP (catalogue No., DY998) manufactured by R&D, the CRTAC1 protein concentration in the EDTA plasma was measured. More specifically, the CRTAC1 protein concentration in the blood plasma derived from cerebral infarction patients (a mixture of several ten patients) was arbitrarily defined as 100 U/mL, and this was used for preparation of a calibration curve. Based on the calibration curve, the relative concentration (U/mL) of the CRTAC1 protein in the blood plasma was calculated.

Subsequently, in order to evaluate the distinguishing performance at each timing of blood collection, the AUC value of the ROC curve was calculated according to the method described in Fawcett, T. (2004) ROC Graphs: Notes and Practical Considerations for Researchers. The results are shown in Table 2. A test method is shown to be useful for distinguishing between a cerebral infarction patient and a healthy individual if the value is higher than 0.7 at each timing of blood collection. That is, it was found that a test for distinguishing between a healthy individual and a cerebral infarction patient can be carried out by obtaining blood from a cerebral infarction patient immediately after the onset and 7 days after the onset, and comparing the plasma concentrations of the CRTAC1 protein with those of healthy individuals.

**[Table 2]**

| | DAY0 | DAY7 |
|---|---|---|
| Group 1 | Cerebral infarction | Cerebral infarction |
| Group2 | Healthy | Healthy |
| AUC of ROC curve | 0.794 | 0.880 |
| Number of group 1 | 91 | 102 |
| Number of group 2 | 71 | 71 |

### Example 2

### Evaluation of Test Method for Distinguishing among Disease Types of Cerebral Infarction Using CRTAC1 Protein Marker

Blood was collected immediately after the onset (indicated as "DAY 0" in the table) and 7 days after the onset (indicated as "DAY 7" in the table) of cerebral infarction from patients (see Table 3 for the number of individuals) who were definitely diagnosed with the respective disease types of cerebral infarction, that is, "lacunar infarction" (which is referred to as "lacunar" in the table), "atherothrombotic cerebral infarction" (which is referred to as "atherothrombotic" in the table), "cardiogenic brain embolism" (which is referred to as "cardiogenic brain embolus" in the table), "aortogenic brain embolism" (which is referred to as "aortogenic brain embolus" in the table), "branch atheromatous disease" (which is referred to as "BAD" in the table), arterial dissection (which is referred to as "arterial dissection" in the table), and disease types that cannot be categorized into any of these (which are referred to as "unclassified type" in the table); and EDTA plasma was obtained from each blood sample.
The plasma concentration of the CRTAC1 protein was measured in the same manner as in the method in Example 1, and the measured value was used to calculate the AUC value of the ROC curve in the same manner as in Example 1.
A test method is shown to be useful for specifying the disease type of a cerebral infarction patient if the value is higher than 0.7 at each timing of blood collection.

That is, it was found that, by comparing the concentrations of the CRTAC1 protein in blood plasma of patients suffering from the respective disease types collected immediately after the onset, a test can be carried out for distinguishing between cerebral infarction patients with the disease types of "atherothrombotic cerebral infarction" and "arterial dissection", and, by similar comparison, tests can be carried out for distinguishing between cerebral infarction patients with the disease types of "cardiogenic brain embolism" and "arterial dissection", distinguishing between cerebral infarction patients with the disease types of "lacunar infarction" and "Branch atheromatous disease", distinguishing between cerebral infarction patients with the disease types of "lacunar infarction" and "arterial dissection", distinguishing between cerebral infarction patients with the disease types of "aortogenic brain embolism" and "Branch atheromatous disease", distinguishing between cerebral infarction patients with the disease types of "aortogenic brain embolism" and "arterial dissection", distinguishing between cerebral infarction patients with the disease types of "Branch atheromatous disease" and "arterial dissection", distinguishing between cerebral infarction patients with the disease types of "Branch atheromatous disease" and "unclassified type cerebral infarction", distinguishing between cerebral infarction patients with the disease types of "cardiogenic brain embolism" and "Branch atheromatous disease", distinguishing between cerebral infarction patients with the disease types of "atherothrombotic cerebral infarction" and "Branch atheromatous disease", distinguishing between cerebral infarction patients with the disease type of "Branch atheromatous disease" and other disease types, and distinguishing between cerebral infarction patients with the disease type of "arterial dissection" and other disease types.

Further, it was found that, by comparing the concentration of the CRTAC1 protein in blood plasma of patients suffering from the respective disease types collected 7 days after the onset, a test can be carried out for distinguishing between cerebral infarction patients with the disease types of "atherothrombotic cerebral infarction" and "lacunar infarction", and, by similar comparison, tests can be carried out for distinguishing between cerebral infarction patients with the disease types of "cardiogenic brain embolism" and "lacunar infarction", distinguishing between cerebral infarction patients with the disease types of "cardiogenic brain embolism" and "Branch atheromatous disease", and distinguishing between cerebral infarction patients with the disease types of "atherothrombotic cerebral infarction" and "Branch atheromatous disease".

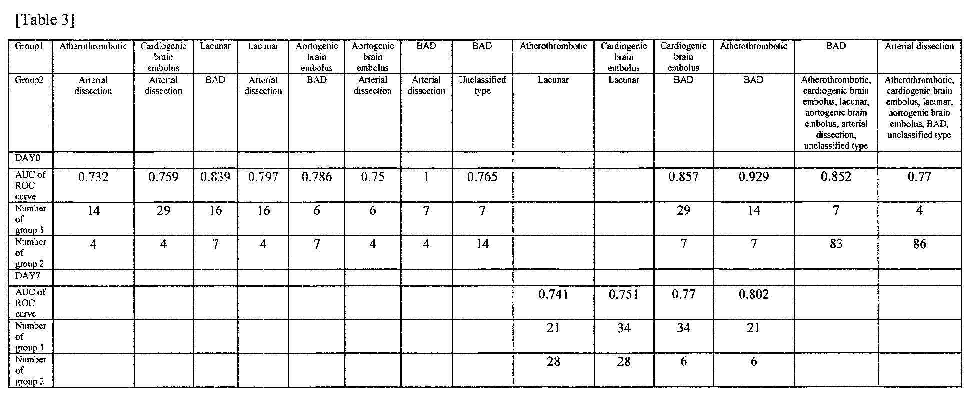

### Example 3

### Evaluation of Test Method for Prediction of Prognosis of Cerebral Infarction Using CRTAC1 Protein Marker

The prognosis of cerebral infarction is indicated after a certain period of time following the onset of cerebral infarction in terms of the degree of disability due to cerebral infarction in the patient. Patients were classified based on the degree of disability determined 3 months after the onset of cerebral infarction using the Japanese version of the modified Rankin Scale (mRS) criterion (Yukito Shinohara et al., mRS Reliability Study Group, Reliability of modified Rankin Scale-Introduction of a guidance scheme and a questionnaire written in Japanese-, Jpn J Stroke 2007, 29:6-13) into 0 to 5, and EDTA plasma was obtained from blood that was collected from the patients (see Table 4 for the number of individuals) 7 days after the onset of cerebral infarction (indicated as "DAY7" in the table).
The plasma concentration of the CRTAC1 protein was measured in the same manner as in the method in Example 1, and the measured value was used to calculate the AUC value of the ROC curve in the same manner as in Example 1. The results are shown in Table 4.
A test method is shown to be useful for predicting the prognosis of a cerebral infarction patient if the value is higher than 0.7 at each timing of blood collection.

**[Table 4]**

| Group 1 | 5,4,3,2 | 5, 4, 3 | 5, 4 |
|---|---|---|---|
| Group2 | 1, 0 | 2, 1, 0 | 3, 2, 1, 0 |
| DAY7 | | | |
| AUC of ROC curve | 0.7 | 0.73 | 0.722 |
| Number of group 1 | 43 | 23 | 14 |
| Number of group 2 | 55 | 75 | 84 |

More specifically, in terms of the degree of disability evaluated 3 months after the onset of cerebral infarction (prognosis), it was found that, by comparing the concentrations of the CRTAC1 protein in blood plasma collected 7 days after the onset from patients who were classified into the respective ranks 3 month after the onset, a test can be carried out for distinguishing between a patient ranked from 2 to 5 and a patient ranked from 0 to 1; between a patient ranked from 3 to 5 and a patient ranked from 0 to 2; and between a patient ranked from 4 to 5 and a patient ranked from 0 to 3; in terms of the rank evaluated 3 months after the onset.

### Example 4

### Evaluation of Test Method for Prediction of Progressiveness of Cerebral Infarction Using CRTAC1 Protein Marker

In cases where NIHSS at any of the timings of 3 days after the onset, 7 days after the onset and 14 days after the onset was higher by 1 or more points than NIHSS evaluated immediately after the onset of cerebral infarction (indicated as "DAY0" in the table), the cerebral infarction was defined as being progressive, while in cases where NIHSS did not change or decreased by 1 or more points, the cerebral infarction was defined as being nonprogressive. In order to eliminate bias among the subgroups in terms of the severity and the disease type, patients with NIHSS of not less than 15 were excluded. The plasma concentration of the CRTAC1 protein was measured in the same manner as in the method in Example 1, and the measured value was used to calculate the AUC value of the ROC curve in the same manner as in Example 1. The results are shown in Table 5.
A test method is shown to be useful for distinguishing between a patient with progressive cerebral infarction and a patient with nonprogressive cerebral infarction if the value is higher than 0.7 at the timing of blood collection 7 days after the onset. That is, it was found that, by obtaining blood from cerebral infarction patients 7 days after the onset and comparing the plasma concentrations of the CRTAC1 protein, a test can be carried out for distinguishing between a patient with progressive cerebral infarction and a patient with nonprogressive cerebral infarction.

**[Table 5]**

| Group 1 | Progressive |
|---|---|
| Group2 | Nonprogressive |
| DAY7 | |
| AUC of ROC curve | 0.808 |
| Number of group 1 | 12 |
| Number of group2 | 78 |

### INDUSTRIAL APPLICABILITY

By the method of the present invention, it is possible to carry out diagnosis of cerebral infarction, a test for prediction of the prognosis of cerebral infarction, and evaluation of a therapeutic or prophylactic effect on cerebral infarction. Thus, the method of the present invention is useful in the fields of medical care and diagnosis. Further, by the method of the present invention, a therapeutic or prophylactic drug for cerebral infarction can be screened. Thus, the method of the present invention is also useful in the field of pharmaceuticals.

## Claims

1. A test method for diagnosing cerebral infarction in a subject animal, comprising the step of measuring the amount of cartilage acidic protein 1 in a blood sample collected from said animal, wherein, in cases where the amount of said protein is lower than the amount in a healthy individual, said animal is judged to be cerebral infarction.

2. The test method for diagnosing cerebral infarction according to claim 1, wherein said blood sample collected from said subject animal is a blood sample collected from said subject animal at any period between immediately after onset and 20 days after onset of cerebral infarction-like symptoms.

3. A test method for diagnosing the disease type of cerebral infarction in a subject animal, said method comprising the step of measuring the amount of cartilage acidic protein 1 in a blood sample collected from said animal.

4. The test method according to claim 3, wherein said diagnosis of the disease type is for diagnosing any of lacunar infarction, atherothrombotic, cardiogenic brain embolism, aortogenic brain embolism, branch atheromatous disease, arterial dissection and unclassified type.

5. A test method for predicting prognosis of cerebral infarction in a subject animal, said method comprising the step of measuring the amount of cartilage acidic protein 1 in a blood sample collected from said animal.

6. The test method for predicting prognosis of cerebral infarction according to claim 5, wherein said blood sample collected from said subject animal is a blood sample collected from said subject animal 7 days after onset of cerebral infarction.

7. A test method for predicting progressiveness of cerebral infarction in a subject animal, said method comprising the step of measuring the amount of cartilage acidic protein 1 in a blood sample collected from said animal.

8. The test method for predicting progressiveness of cerebral infarction according to claim 7, wherein said blood sample collected from said subject animal is a blood sample collected from said subject animal 7 days after onset of cerebral infarction.

9. A method for evaluating a prophylactic or therapeutic effect on cerebral infarction in an animal in need of prophylaxis or therapy of cerebral infarction, said method comprising the step of measuring the amount of cartilage acidic protein 1 in a blood sample collected from said animal to which a prophylactic drug or therapeutic drug for cerebral infarction was administered.

10. A method for screening a prophylactic drug or therapeutic drug for cerebral infarction, said method comprising the step of contacting a subject with a candidate compound for prophylactic drug or therapeutic drug for cerebral infarction and then measuring the amount of cartilage acidic protein 1 in said subject.

11. A kit for testing for cerebral infarction, for evaluating a therapeutic effect on cerebral infarction, or for screening a candidate compound for a prophylactic/therapeutic drug for cerebral infarction, said kit comprising a reagent which enables measurement of the amount of cartilage acidic protein 1.
